# EUROPEAN PATENT SPECIFICATION

(11) **EP 2 532 308 B1**
(45) Date of publication and mention of the grant of the patent: **30.05.2018**
(21) Application number: 11169170.5
(22) Date of filing: 08.06.2011
(51) Int. Cl.: A61B 17/00, A61B 17/29, A61B 17/32

(54) **Medical instrument**
Medizinisches Instrument
Instrument médical

(43) Date of publication of application: 12.12.2012
(73) Proprietor: The University of Dundee, Dundee DD1 4HN (GB)
(72) Inventor: Coleman, Stuart, Dundee DD2 1FD (GB)
(74) Representative: Witte, Weller & Partner Patentanwälte mbB

(56) References cited:
- DE-U1- 9 404 423
- US-A1- 2010 234 687

## Description

The invention relates to a medical instrument according to the preamble of claim 1.

A medical instrument of the kind mentioned before is known from US 2010/0234687 A1. The preamble of claim 1 is based on this document.

A medical instrument according to the present invention can be designed as an instrument for grasping tissue, as an instrument for cutting tissue, as an instrument for use in electro surgery (coagulation and cutting with high frequency current), as a needle holder, a dissector, for example.

A medical instrument according to the invention is particularly designed for use in endoscopic surgery.

In endoscopic surgery medical instruments are required which have a shaft which has at least one bent portion between the distal end and the proximal end. Due to the at least one bent portion, the working part arranged at the distal end can lie outside of the longitudinal center axis of the proximal end of the shaft. In combination with the rotatability of the shaft about its longitudinal axis, the working part can be brought to different locations in the body cavity simply by rotating the shaft without displacing the instrument as a whole. Simple rotation of the shaft about its longitudinal axis allows a full 360° range of direction control of the working part of the instrument.

There are several types of bents which differ in the number of curvatures along the longitudinal axis of the shaft, the most important of which are the so called distal arc having one curvature near the distal end of the shaft, and the bayonet type having two counter-directed curvatures near the distal end so that the distal end of the shaft is approximately parallel to the proximal end of the shaft, but lies outside the longitudinal axis of the proximal end of the shaft.

It is to be understood in the context of the present invention that the bent portion of the shaft can be permanent and unchangeable, but it is also possible within the scope of the present invention that the at least one bent portion can be made by the user of the instrument by bending the shaft by hand and can be changed according to the needs of the surgery. Further, the term "bent portion" can also include a joint or articulation.

A medical instrument known from the DE-catalogue "THE WORLD OF ENDOSCOPY", vol. "LAPAROSCOPY", published in 2002, edited by company Karl Storz GmbH & Co. KG, pages DGC 55 - DGC 56 mentioned at the outset has an elongated shaft which can have a bent portion between the distal end and the proximal end, and which is rotatable relative to the handle about a longitudinal axis of the shaft. The working part arranged at the distal end of the shaft is rotatably fixed with respect to the shaft and, thus, when the shaft is rotated, the angular orientation of the working part with respect to the handle is altered according to the angle of rotation of the shaft.

The working part of the known instrument has two jaw parts one of which is movable and the other one immovable. The movable jaw part is connected to the distal end of an elongated element extending through the shaft and connected to a movable grip part of the handle. The force transmission element is movable relative to the shaft in direction of the longitudinal axis, and acts to actuate the movable jaw part by actuating the movable grip part.

Another medical instrument is known from document WO 2006/100658 A2. This known medical instrument has a shaft having at least one bent portion. The shaft of this known medical instrument is not rotatable about the longitudinal axis relative to the shaft, but the shaft can only be rotated by rotating the handle. Instead, the working part at the distal end of the shaft can be rotated relative to the shaft and handle by operating an operating element arranged at the handle.

This medical instrument has the disadvantage that the handle must be rotated in order to rotate the shaft which is particularly inconvenient if the handle is configured in scissors-like fashion. Rotation of the handle requires wrist rotation which significantly increases fatigue when using this instrument in a long-lasting surgery.

EP 1 872 729 B1 discloses a medical instrument for grasping an object, in particular a needle holder, the working part of which has a multitude of degrees of freedom. The working part is as well rotatable with respect to the handle as well as to the shaft, and the shaft is also rotatable with respect to the handle and with respect to the working part. This medical instrument is comparatively sophisticated and as a result, cost intensive.
US 2010/234687 A1 mentioned above discloses a medical instrument comprising an elongated shaft having a distal end and a proximal end and at least one bent portion between the distal end and the proximal end and comprising an end effector being arranged at the distal end of the shaft. The end effector is connected to the handle through an actuating element. The handle is rotationally connected with the proximal end of the shaft and the end effector is rotationally connected with the distal end of the shaft. The handle and the end effector are connected to each other such that by rotating the handle with respect to the shaft a rotation of the end effector relative to the shaft is effected.
DE 94 04 423 U1 discloses a surgical instrument, especially an instrument for grasping and/or cutting tissue comprising an end effector being mounted at the distal end of an at least partially bent outer shaft tube and comprising a handle being mounted at the proximal end of the shaft for actuating the end effector. Through a radial flexible metal tubing, which is guided within the tubular shaft, a force transmission element is guided to transmit manipulations being effected at the handle to the end effector. The end effector is rotationally mounted at the distal end of the tubular shaft to transmit manipulations from the proximal end of the tubular shaft to the end effector through the flexible metal tubing. The flexible metal tubing ends at the proximal end at a steel tubing comprising a four-cornered shaft. The four-cornered shaft is connected to a rotating element being rotationally mounted at the handle.

It is an object of the invention to improve a medical instrument of the type mentioned at the outset such that the number of degrees of freedom of the working part is increased with respect to the medical instrument known from US 2010/0234687 A1 mentioned at the outset without increasing constructional expenditure.

According to the invention, the afore-mentioned object is achieved with respect to the medical instrument of the type mentioned at the outset by the characterizing features of claim 1.

The medical instrument according to the invention has the working part rotationally fixed relative to the handle, while the shaft is rotatable relative to the handle and to the working part. Further, the elongated element of the instrument according to the invention is rotationally fixed relative to the handle and the shaft is rotatable relative to the elongated element.

The medical instrument according to the invention has the advantage that the working part remains in a fixed orientation relative to the handle while the instrument shaft is rotated. This configuration has the advantage in certain surgical procedures, for example when tissue must be tensioned to facilitate cutting. This may be achieved with the medical instrument according to the invention by grasping the tissue and rotating the angled instrument shaft so that the working part is laterally displaced while the working part remains in its original angular orientation with respect to the handle. Thus, it is prevented that the tissue is wrapped around the working part when rotating the shaft. By rotating the shaft by 360° about the longitudinal axis, the working part describes a trajectory which is a circle about the longitudinal axis of the shaft while the angular orientation of the working part with respect to its own longitudinal axis does not alter along the circular path.

The elongated element is configured for rotationally fixed connection with the handle in at least two different angular orientations about the longitudinal axis.

This configuration has the advantage that the working part can be rotationally fixed relative to the handle in at least two different angular orientations about the longitudinal axis. Different angular orientations of the working part are advantageous, in particular in the case that the working part comprises jaw parts which can be opened and closed. Thus, the plane of the closing and opening movement of the jaw parts can be differently set with respect to the handle in this refinement. This configuration advantageously reduces the need for a plurality of instruments having working parts in different angular orientations about the longitudinal axis with respect to the handle.

The elongated element has a connecting portion for connection with the handle, and the handle has a receiving portion, into which the connecting portion is inserted and held rotationally fixed with respect to the handle.

This measure has the advantage of a structurally simple connection of the proximal end of the elongated element with the handle. In addition, the connecting portion can be configured to provide an axially fixed connection of the elongated element with the handle and, thus, to axially fix the working part relative to the handle.

In a refinement, it is further preferred if the connecting portion is insertable into and removable from the receiving portion in direction transverse to the longitudinal axis.

This measure is particularly advantageous in connection with the refinement mentioned above according to which the elongated element is configured for rotationally fixed connection with the handle in at least two different angular orientations about the longitudinal axis. By virtue of the present refinement, the desired angular orientation of the working part relative to the handle can be set by simply removing the receiving portion in direction transverse to the longitudinal axis and to turn the elongated element, and thereby, the working part into the desired angular orientation and to reinsert the connecting portion into the receiving portion for rotationally fixing the elongated element with respect to the handle. A tool for carrying out the afore-mentioned manipulations in order to alter the angular orientation of the working part is not necessary so that the user of the instrument can set the angular orientation of the working part during a surgical procedure from his or her own end. Further, the removal/reinsertion of the elongated element into the handle can be achieved in the same way as described when the instrument is disassembled/assembled to allow for cleaning and sterilization, so an additional mechanism is not required.

It is to be understood that the connecting portion can be secured in the receiving portion in that the connecting portion is cliqued or latched in the receiving portion in order to ensure that the connecting portion is not disengaged from the receiving portion in an unintentional manner.

In a further preferred refinement, the connecting portion of the elongated element has a cross-section transverse to the longitudinal axis which is polygonal in shape, and the receiving portion has a cross-section which is polygonal in shape.

This measure has the advantage to provide a mechanism for a rotationally fixed connection of the elongated element with the handle which is constructionally simple. A further advantage of this measure is that the polygonal shape of the cross-section of the connecting portion and the polygonal shape of the cross-section of the receiving portion allow for different angular orientations about the longitudinal axis of the elongated element and, thus, the working part with respect to the handle. The polygonal shape of the cross-section of the connecting portion and the polygonal shape of the receiving portion provide a simple rotationally fixed connection by a form-fit.

It is preferred in connection with the afore-mentioned refinement if the polygonal shape of the cross-section of the connecting portion has an even number of edges, and wherein the polygonal shape of the cross-section of the receiving portion is rectangular.

Due to the even number of edges of the cross-section of the connecting portion and the rectangular cross-section of the receiving portion, the connecting portion can be inserted in different angular orientations about the longitudinal axis into the receiving portion, wherein always two parallel edges of the connecting portion are held between two parallel edges of the receiving portion.

Accordingly, the cross-section of the connecting portion can be rectangular so that the elongated element can be inserted in four different angular orientations, or the cross-section of the connecting portion can be hexagonal providing six different angular orientations of the working part with respect to the handle, or octagonal providing eight different angular orientations, etc.

The present invention cannot only be used in medical instruments with an immovable working part, but also in medical instruments the working part of which has at least one movable working element, and the handle of which has at least one movable grip part, wherein the elongated element is a force transmission element which is movable with respect to the shaft in direction of the longitudinal axis and operatively connected to the at least one movable working element and the at least one movable grip part.

In the afore-mentioned preferred refinement, the medical instrument according to the invention can be configured as a grasping or cutting instrument wherein in the case of a grasping instrument tissue can be grasped by the working elements or in case of a cutting instrument, tissue can be cut by the working elements.

In a further preferred refinement, a proximal end of the working part is connected to the distal end of the shaft such that the working part is immovable with respect to the shaft in direction of the longitudinal axis, but the shaft is rotatable about the longitudinal axis relative to the working part.

This measure has the advantage that the working part is axially fixed with respect to the shaft thus preventing an axial displacement of the working part relative to the shaft which is, for example, advantageous if the instrument is a grasping or retracting instrument and the working part is used to retract or tension tissue.

In connection with the afore-mentioned refinement, it is further preferred if a proximal end of the working part has a first radial shoulder extending in circumferential direction, the distal end of the shaft has a second radial shoulder extending in circumferential direction, wherein the first shoulder and the second shoulder are substantially complementary with respect to one another and engage with one another.

This refinement provides a constructionally simple connection mechanism for connecting the working part with the shaft such that the working part and the shaft are axially fixed with respect to one another on the one hand, and on the other hand, the shaft remains rotatable with respect to the working part. The first shoulder and the second shoulder which engage with one another provide the axial fixation, while the first shoulder and the second shoulder are slidingly rotatable with respect to one another in order to provide the relative rotational movement between the working part and the shaft.

Further features and advantages will be apparent from the following description and the drawings.

It is to be understood that the afore-mentioned features and the features described hereinafter are not only applicable in the given combination, but also in other combinations or in isolation without departing from the scope of the present invention.

An exemplary embodiment is shown in the drawings and will be described hereinafter with respect thereto. In the drawings:
- Fig. 1: shows a medical instrument in a side view;
- Fig. 2: shows the instrument of Fig. 1 in a side view and in enlarged scale, wherein a shaft of the instrument is shown partially;
- Fig. 3: shows the instrument of Figs. 1 and 2 in the same side view as in Fig. 2, wherein the shaft of the instrument has been rotated by 180° about the longitudinal axis of the shaft;
- Fig. 4: shows a distal end portion of the instrument in Figs. 1 through 3 in a further enlarged scale in a side view;
- Fig. 5: shows the distal end portion of Fig. 4 in a longitudinal section;
- Fig. 6: shows a portion of the instrument in Figs. 1 through 3 in the region of a proximal end of an elongated element of the instrument and of a portion of a handle of the instrument; and
- Fig. 7: shows a sectional view taken along the line VII-VII in Fig. 6.

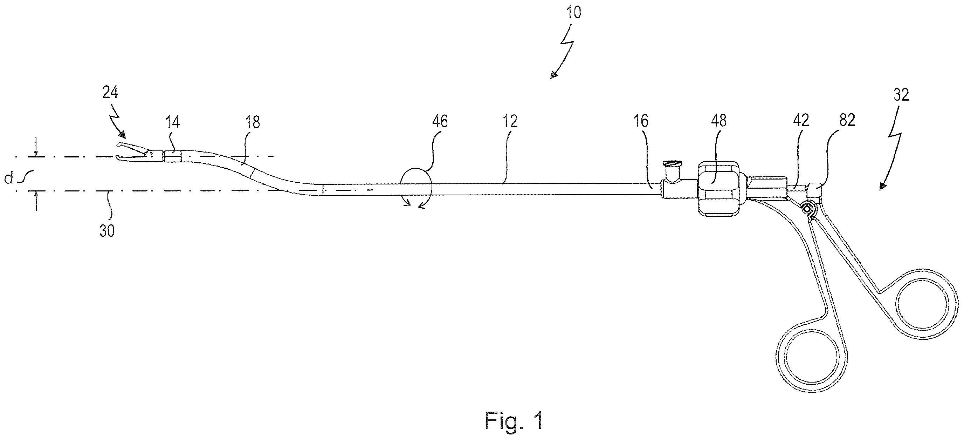

Figs. 1 through 3 show a medical instrument labelled with general reference numeral 10. The medical instrument 10 is configured as a forceps, in particular for use in endoscopic surgery.

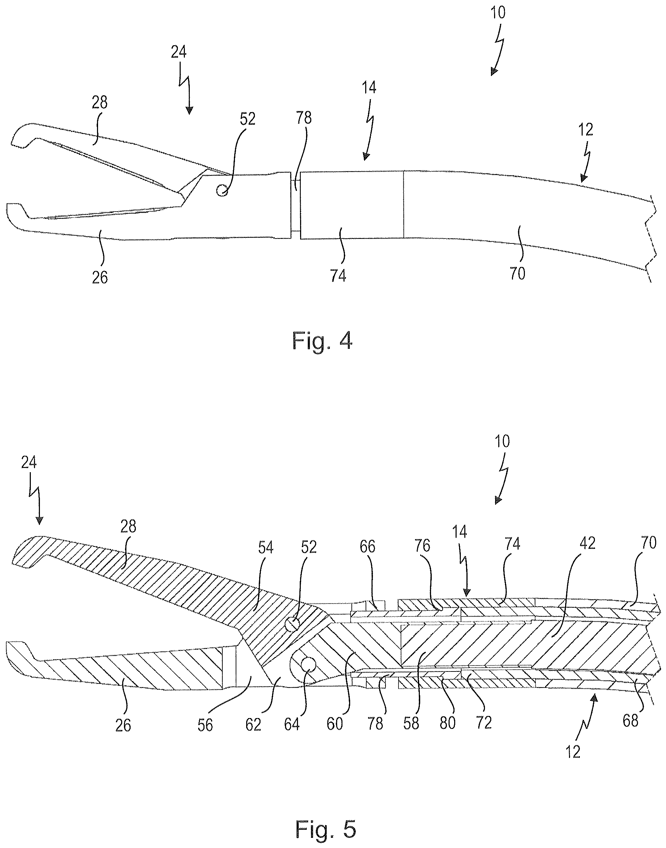

Further details of the instrument 10 are shown in Figs. 4 through 7.

With reference to Figs. 1 through 3, the instrument 10 comprises an elongated shaft 12 which has a distal end 14 and a proximal end 16.

The shaft 12 has at least one bent portion 18 between the distal end 14 and the proximal end 16. In the present embodiment, the bent portion 18 is a so-called bayonet bent portion having two curvatures 20 and 22 having their center of curvature on opposite sides of the shaft 12. Thus, the bent portion 18 resembles an S-shape. The distal end 14 and the proximal end 16 are substantially parallel with one another, but offset with respect to one another.

It is to be understood that the bent portion 18 could also have only one curvature, for example the curvature 22 so that in this case the distal end 14 and the proximal end 16 form an angle with one another. It is also conceivable that the shaft 12 has more than one bent portion.

The bent portion 18 may be permanent if the shaft 12 is rigid, wherein the bent portion 18 has been shaped when manufacturing the shaft 12. It is, however, also possible that the shaft 12 is semi-rigid or semi-flexible so that the bent portion 18 can be shaped by the user of the instrument 10 by bending the shaft 12 into a desired shape.

Furthermore, the term "bent portion" according to the invention can also encompass a configuration of the shaft 12 where two portions of the shaft are articulated with one another via a joint.

A working part 24 is arranged at the distal end 14 of the shaft. The working part 24 has an immovable working element 26 and a movable working element 28. The working elements 26 and 28 are configured as jaw parts for grasping tissue, for example. In the embodiment shown, the medical instrument 10, thus, is a grasping instrument.

Due to the bayonet bent portion 18, the working part 24 is laterally displaced with respect to a longitudinal axis 30 of the shaft 12 (the longitudinal axis 30 coincides with the longitudinal axis of the proximal end 16) by a distance d (see Fig. 1).

A handle 32 is arranged at the proximal end 16 of the shaft 12. The handle 32 has an immovable grip part 34 and a movable grip part 36. The immovable grip part 34 has a finger ring 38, and the movable grip part 36 has a finger ring 40. The movable grip part 36 is connected with the immovable grip part 34 via a joint or hinge 37.

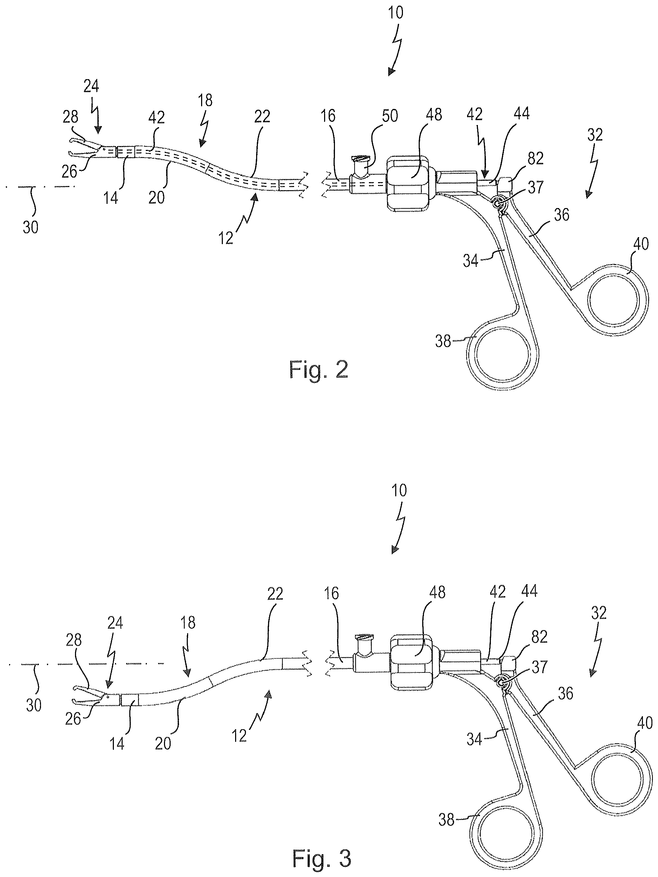

The movable grip part 36 is operatively connected with the movable working element 28 via an elongated element 42 (shown in Fig. 2 by broken lines), wherein the elongated element 42 extends through the shaft 12 (see Fig. 5) so that only a proximal end 44 can be seen in Figs. 1 through 3. The connection of the elongated element 42 with the movable working element 28 on the one hand and with the movable grip part 36 on the other hand will be described later.

The elongated element 42 is configured as a force transmission element in the embodiment shown and is axially movable with respect to the shaft 12 in order to transfer a movement of the movable grip part 36 into a movement of the movable working element 28 for closing and opening the movable working element 28.

The elongated element 42 is sufficiently flexible in order to be able to conform to the bent portion 18 of the shaft 12, but is sufficiently rigid in order to be able to transfer push and pulling forces from the movable grip part 36 to the movable working element 28. Further, the elongated element 42 must also have sufficient torsional rigidity to constrain the rotation of the working part 24 about its longitudinal axis.

The shaft 12 is rotatable about the longitudinal axis 30 as indicated by an arrow 46 in Fig. 1. The shaft 12 is rotatable in both rotational directions over 360°. An actuating element 48 is arranged distally from the immovable grip part 34 for actuating the rotation of the shaft 12. The actuating element 48 is configured as a wheel which can be actuated by the index finger while the finger rings 38 and 40 are held with the thumb and the middle finger of the same hand holding the instrument 10.

Distally of the actuating element 48, there is a connector 50 for connecting an irrigation and/or suction pipe to the instrument 10. The connector 50 is fixedly connected with the shaft 12. Alternatively, the connector 50 can be fixedly connected with the handle 32 and, thus, does not rotate together with the shaft 12 relative to the handle 32, when the shaft 12 is rotated.

While the shaft 12 is rotatable with respect to the handle 32, the working part 24 and the elongated element 42 are not rotatable with respect to the handle 32 about their own longitudinal axes, but they are rotationally fixed relative to the handle 32. Further, the shaft 12 is rotatable relative to the working part 24, and the elongated element 42 is rotationally fixed with respect to the working part 24.

It will be described hereinafter, how the rotatability of the shaft 12 relative to the working part 24 is accomplished, while the working part 24 is not rotatable relative to the handle 32.

With reference to Figs. 4 and 5, the movable working element 28 is hingedly connected with the immovable working element 26 via a hinge 52 which is formed by a pin extending through the immovable working element 26 and the movable working element 28. A proximal end 54 of the movable working element 28 is received within a clevis 56 of the immovable working element 26. A distal end 58 of the elongated element 42 (force transmission element) has a link 60 which is received in a clevis 62 of the movable working element 28 and connected thereto via a hinge 64 formed by a pin, spaced apart from the hinge 52. By movement of the elongated element 42 in proximal direction, the movable working element 28 is, thus, pivoted about the hinge 52 towards the immovable working element 26 so that the jaw parts are closed, and by a movement of the elongated element 42 in distal direction the movable working element 28 is pivoted away from the immovable working element 26 and the jaw parts are opened.

The working part 24 has a proximal end 66 which forms the proximal end of the immovable working element 26 in the present embodiment, and which is connected to the distal end 14 of the shaft 12 such that the working part 24 is axially fixed with respect to the shaft 12, but the shaft 12 is rotatable relative to the working part 24, as described hereinafter.

The shaft 12 comprises a tube 68, for example a metal tube, through which the elongated element 42 extends. In the present embodiment, the tube 68 is covered with an insulating sheath 70, for example a polymer material shrinked onto the tube 68.

A distal end 72 of the tube 68 is fixedly connected with a sleeve 74, for example by welding, soldering or threading. The sleeve 74 forms the distal end 14 of the shaft 12. The sleeve 74 may optionally be removable from the tube 68 in order to facilitate the removal of the elongated element 28 and the working part 24 from the instrument shaft 12, in order to allow cleaning and maintenance of the instrument more easily.

The sleeve 74 has a shoulder 76 extending in circumferential direction about the longitudinal axis of the distal end 14 of the shaft 12 and directed radially inwardly with respect to the longitudinal axis.

The proximal end 66 of the working part 24 is fixedly connected, for example by welding, soldering or threading, with another sleeve 78 which extends into the sleeve 74 and has a shoulder 80 extending in circumferential direction about the longitudinal axis of the distal end 14 of the shaft 12 and directed radially outwardly with respect to the longitudinal axis.

As shown in Fig. 5, the shoulders 76 and 80 are substantially complementary with respect to one another and engage with one another. The sleeve 74 is not fixedly connected with the sleeve 78, but the sleeve 74 can rotationally slide about the longitudinal axis of the distal end 14 of the shaft 12 relative to the sleeve 78, while an axial movement of the sleeve 78 relative to the sleeve 74 is prevented.

The sleeve 78 which is fixedly connected with the proximal end 66 of the working part 24 abuts against the distal end 72 of the tube 68 and, thus, it is axially immovably held between the shoulder 76 of the sleeve 74 and the distal end 72 of the tube 68. Thus, the working part 24 is axially immovably connected with the shaft 12, while the shaft 12 can rotate about the longitudinal axis with respect to the working part 24.

Assembling the working part 24 and the shaft 12 can be carried out as follows. First, the two working elements 26 and 28 are joined together with the pin forming the hinge 52. The link 60 of the elongated element 42 is then connected to the movable working element 28 with a pin to form the hinge 64, wherein the elongated element 42 is fixed to the link 60 in case that the link 60 is not made in one piece with the remaining body of the elongated element 42. In the next step, the sleeve 78 is inserted into the proximal end of the sleeve 74 and pushed through the sleeve 74 until the shoulders 76 and 80 abut against one another. The distal end of the sleeve 78 is then fixed to the proximal end 66 of the immovable working element 26. The elongated element 42 is then inserted into the shaft 12, i.e. into tube 68 and the sleeve 74 is then fixed to the tube 68 whereby the sleeve 78 and, thus, the working part 24 is trapped in position. The fits of the parts described before should ensure that the working part 24 is not overly loose while the shaft 12 remains freely rotatable with respect to the working part 24.

As the elongated element 42 is rotationally fixed to the working part 24 via the hinge 64, it is evident that the shaft 12 is not only free rotatable with respect to the working part 24, but also with respect to the elongated element 42. The elongated element 42 can be rotationally fixed to the working part 24 both via the hinge 64 and because the distal end of the elongated element 42 has flat sides and is located within the clevis 62, which prevents its rotation about its longitudinal axis.

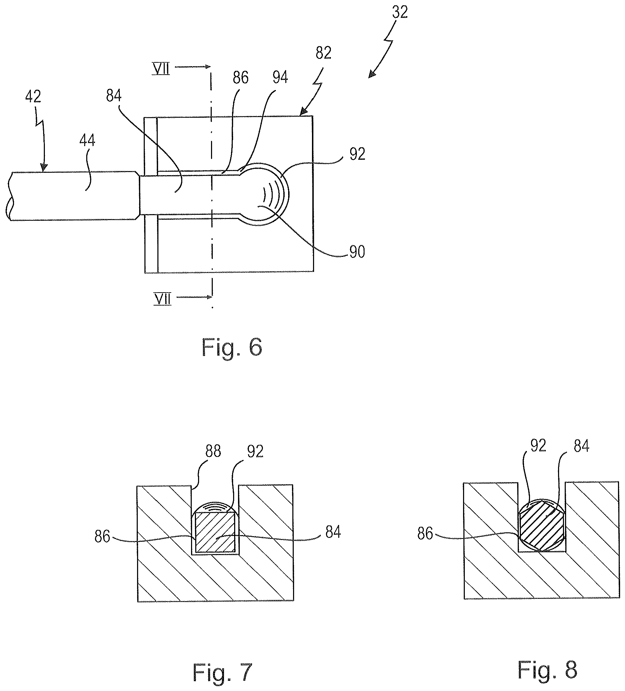

With reference to Figs. 6 and 7, the rotationally fixed connection of the elongated element 42 with the handle will be described.

Fig. 6 shows the proximal end 44 of the elongated element 42 and a portion 82 of the handle 32. The portion 82 is an integral part of the movable grip part 36.

The elongated element 42 has, at the proximal end 44, a connecting portion 84 for connection with the handle 32, i.e. with the movable grip part 36. The connecting portion 84 is inserted in a receiving portion 86 which is, in the present embodiment, configured as a groove in the portion 82 of the handle 32. The receiving portion 86 is open at an end 88 so that the connecting portion 84 can be inserted into the receiving portion 86 and removed from the receiving portion 86 in direction transverse to the longitudinal axis of the elongated element 42.

The connecting portion is held rotationally fixed in the receiving portion 86 in order to prevent a rotation of the elongated element 42 and, thus, the working part 24 relative to the handle 32 about the longitudinal axis of the elongated element 42.

In the embodiment shown, the connecting portion 84 has a cross-section which is rectangular, and the receiving portion 86 has a cross-section which is rectangular, too. By this arrangement, the connecting portion 84 can be inserted into the receiving portion 86 in four different angular orientations about the longitudinal axis of the shaft with respect to the handle 32. Hence, it is possible to have four different angular orientations of the working part 24 with respect to the handle 32. While Fig. 2 shows an angular orientation of the working part 24 with respect to the handle 32 where the working elements 26 and 28 lie in a plane parallel to the plane of the grip parts 34 and 36 and the movable working element 28 forms the upper working element, it is also possible to insert the connecting portion 84 into the receiving portion 86 such that the working elements 26 and 28 lie in a plane perpendicular to the plane of the grip parts 34, 36, with the movable working element 28 forming the left working element or the right working element, and it is further possible to insert the connecting portion 84 into the receiving portion 86 such that the working elements 26 and 28 again lie in the same plane as the grip parts 34, 36, but the movable working element 28 forms the lower working element.

The connecting portion 84 of the elongated element 42 is adjacent to an enlarged portion 90 which is enlarged in direction transverse to the longitudinal axis of the elongated element 42 in order to axially fix the elongated element 42 to the portion 82. The enlarged portion 90 is designed as a ball, which is inserted in a further receiving portion 92 having a neck 94 through which the enlarged portion 90 cannot pass in distal direction of the longitudinal axis of the elongated element 42. It is to be understood that the enlarged portion 90 can have other shapes than a spherical shape, for example a disk-like shape, and can also be integrated in the connecting portion 84, wherein the receiving portion 86 can have a lateral slot-shaped extension in order to receive the enlarged portion 90, for example.

In general, the connecting portion 84 can have a cross-section which has an even number of edges, wherein the cross-section of the receiving portion 86 is rectangular.

Fig. 8 shows a modification of the connecting portion 84 which has a cross-section which is hexagonal so that the elongated element 42 and, thus, the working part 24 can be inserted into the receiving portion 86 in six different angular orientations with respect to the handle 32.

In order to alter the angular orientation of the working part 24 about its longitudinal axis with respect to the handle 32, it is only necessary to remove the connecting portion 84 from the receiving portion 86 through the open end 88 of the receiving portion 86, to rotate the elongated element 42 by the desired angle and to reinsert the receiving portion 86 into the receiving portion 86 whereafter the elongated element 42 and, thus, the working part 24 is rotationally fixed with respect to the handle 32 again. The removal/reinsertion of the elongated element 42 into the handle 32 can be achieved in the same way as described when the instrument 10 is disassembled/assembled to allow for cleaning and sterilization, so that an additional mechanism is not required.

Other types of connecting mechanisms in order to connect the elongated element 42 with the handle 32 in rotationally fixed fashion are conceivable. For example, the connecting portion 84 and the receiving portion 86 may be configured as splines which can mesh at various angular orientations.

With reference to Figs. 2 and 3, the medical instrument 10 can be operated such that the working part 24 can be laterally displaced with respect to the longitudinal axis 30 from a first position to a second position simply by rotating the shaft 12 about the longitudinal axis 30, wherein the angular orientation of the working part 24 about its own longitudinal axis with respect to the handle 32 remains unchanged.

Fig. 2 shows a first position of the working part 24 where the working part 24 lies above the longitudinal axis 30 of the shaft 12. Fig. 3 shows a second position of the working part 24 in which the working part 24 lies below the longitudinal axis 30 of the shaft 12. Starting from the position of the working part 24 in Fig. 2, the position of the working part 24 in Fig. 3 is achieved by rotating the shaft 12 about the longitudinal axis 30 by an angle of 180°. By virtue of the fact that the working part 24 is rotationally fixed with respect to the handle 32 the angular orientation of the working part 24 has not changed from Fig. 2 to Fig. 3 so that the movable working element 28 is the upper working element in Fig. 2 and in Fig. 3 as well.

## Claims

1. A medical instrument, comprising an elongated shaft (12) having a distal end (14), a proximal end (16) and at least one bent portion (18) between the distal end (14) and the proximal end (16), the shaft (12) being rigid, semi-rigid or semi-flexible, a working part (24) arranged at the distal end (14) of the shaft (12), a handle (32) arranged at the proximal end (16) of the shaft (12), the working part (24) being connected to the handle (32) through an elongated element (42) extending through the shaft (12) and having a distal end (58) connected to the working part and a proximal end (44) connected to the handle (32), wherein the shaft (12) is rotatable relative to the handle (32) about a longitudinal axis (30) of the shaft (12), wherein the working part (24) and the elongated element (42) are rotationally fixed relative to the handle (32) and the shaft (12) is rotatable relative to the working part (24) and to the elongated element (42), **characterized in that** the elongated element (42) is removably connected to the handle (32), and configured for rotationally fixed connection with the handle (32) in at least two different angular orientations about the longitudinal axis (30), wherein the elongated element (42) has a connecting portion (84) for connection with the handle (32), and the handle (32) has a receiving portion (86), into which the connecting portion (84) is inserted and held rotationally fixed with respect to the handle (32).

2. The instrument of claim 1, **characterized in that** the connecting portion (84) is insertable into and removable from the receiving portion (86) in direction transverse to the longitudinal axis (30).

3. The instrument of claim 1 or 2, **characterized in that** the connecting portion (84) has a cross-section transverse to the longitudinal axis (30) which is polygonal in shape, and the receiving portion (86) has a cross-section which is polygonal in shape.

4. The instrument of claim 3, **characterized in that** the polygonal shape of the cross-section of the connecting portion (84) has an even number of edges, and wherein the polygonal shape of the cross-section of the receiving portion (86) is rectangular.

5. The instrument of any of claims 1 through 4, **characterized in that** the working part (24) has at least one movable working element (28), the handle (32) has at least one movable grip part (36), and wherein the elongated element (42) is a force transmission element which is movable with respect to the shaft (12) in direction of the longitudinal axis (30) and operatively connected to the at least one movable working element (28) and the at least one movable grip part (36).

6. The instrument of any of claims 1 through 5, **characterized in that** a proximal end (66) of the working part (24) is connected to the distal end (14) of the shaft (12) such that the working part (24) is immovable with respect to the shaft (12) in direction of the longitudinal axis (30), but the shaft (12) is rotatable about the longitudinal axis (30) relative to the working part (24).

7. The instrument of claim 6, **characterized in that** the proximal end (66) of the working part (24) has a first radial shoulder (80) extending in circumferential direction, the distal end (14) of the shaft (12) has a second radial shoulder (76) extending in circumferential direction, wherein the first shoulder (80) and the second shoulder (76) are substantially complementary with respect to one another and engage with one another.

## Patentansprüche

1. Medizinisches Instrument, mit einem langerstreckten Schaft (12), der ein distales Ende (14), ein proximales Ende (16) und zumindest einen gekrümmten Abschnitt (18) zwischen dem distalen Ende (14) und dem proximalen Ende (16) aufweist, wobei der Schaft (12) starr, halb-starr oder halb-flexibel ist, einem Arbeitsteil (24), das an dem distalen Ende (14) des Schaftes (12) angeordnet ist, einem Handgriff (32), der an dem proximalen Ende (16) des Schafts (12) angeordnet ist, wobei das Arbeitsteil (24) mit dem Handgriff (32) durch ein langerstrecktes Element (42) verbunden ist, das sich durch den Schaft (12) erstreckt und ein distales Ende (58), das mit dem Arbeitsteil verbunden ist, und ein proximales Ende (44) aufweist, das mit dem Handgriff (32) verbunden ist, wobei der Schaft (12) relativ zu dem Handgriff (32) um eine Längsachse (30) des Schaftes (12) drehbar ist, wobei das Arbeitsteil (24) und das langerstreckte Element (42) relativ zu dem Handgriff (32) drehfest sind und der Schaft (12) relativ zu dem Arbeitsteil (24) und zu dem langerstreckten Element (42) drehbar ist, **dadurch gekennzeichnet, dass** das langerstreckte Element (42) abnehmbar mit dem Handgriff (32) verbunden und zur drehfesten Verbindung mit dem Handgriff (32) in zumindest zwei unterschiedlichen Winkelorientierungen um die Längsachse (30) ausgebildet ist, wobei das langerstreckte Element (42) einen Verbindungsabschnitt (84) zur Verbindung mit dem Handgriff (32) aufweist, und der Handgriff (32) einen Aufnahmeabschnitt (86) aufweist, in den der Verbindungsabschnitt (84) eingesetzt und bezüglich des Handgriffs (32) drehfest gehalten ist.

2. Instrument nach Anspruch 1, **dadurch gekennzeichnet, dass** der Verbindungsabschnitt (84) in Richtung quer zur Längsachse (30) in den Aufnahmeabschnitt (86) einsetzbar und von diesem abnehmbar ist.

3. Instrument nach Anspruch 1 oder 2, **dadurch gekennzeichnet, dass** der Verbindungsabschnitt (84) einen Querschnitt quer zur Längsachse (30) aufweist, der polygon-förmig ist, und der Aufnahmeabschnitt (86) einen Querschnitt aufweist, der polygon-förmig ist.

4. Instrument nach Anspruch 3, **dadurch gekennzeichnet, dass** die Polygon-Form des Querschnitts des Verbindungsabschnitts (84) eine gerade Anzahl an Kanten aufweist, und wobei die Polygon-Form des Querschnitts des Aufnahmeabschnitts (86) rechteckig ist.

5. Instrument nach einem der Ansprüche 1 bis 4, **dadurch gekennzeichnet, dass** das Arbeitsteil (24) zumindest ein bewegliches Arbeitselement (28) aufweist, wobei der Handgriff (32) zumindest ein bewegliches Griffteil (36) aufweist, und wobei das langerstreckte Element (42) ein Kraftübertragungselement ist, das bezüglich des Schaftes (12) in Richtung der Längsachse (30) beweglich und mit dem zumindest einen beweglichen Arbeitselement (28) und dem zumindest einen beweglichen Griffteil (36) wirkverbunden ist.

6. Instrument nach einem der Ansprüche 1 bis 5, **dadurch gekennzeichnet, dass** ein proximales Ende (66) des Arbeitsteils (24) mit dem distalen Ende (14) des Schaftes (12) verbunden ist, derart, dass das Arbeitsteil (24) bezüglich des Schaftes (12) in Richtung der Längsachse (30) unbeweglich ist, der Schaft (12) jedoch um die Längsachse (30) relativ zu dem Arbeitsteil (24) drehbar ist.

7. Instrument nach Anspruch 6, **dadurch gekennzeichnet, dass** das proximale Ende (66) des Arbeitsteils (24) eine erste radiale Schulter (80) aufweist, die sich in Umfangsrichtung erstreckt, wobei das distale Ende (14) des Schaftes (12) eine zweite radiale Schulter (76) aufweist, die sich in Umfangsrichtung erstreckt, wobei die erste Schulter (80) und die zweite Schulter (76) im Wesentlichen komplementär zueinander sind und miteinander in Eingriff stehen.

## Revendications

1. Instrument médical, comprenant une tige allongée (12) ayant une extrémité distale (14), une extrémité proximale (16) et au moins une partie courbée (18) entre l'extrémité distale (14) et l'extrémité proximale (16), la tige (12) étant rigide, semi-rigide ou semi-flexible, une partie de travail (24) agencée au niveau de l'extrémité distale (14) de la tige (12), une poignée (32) agencée au niveau de l'extrémité proximale (16) de la tige (12), la partie de travail (24) étant reliée à la poignée (32) par l'intermédiaire d'un élément allongé (42) s'étendant à travers la tige (12) et ayant une extrémité distale (58) reliée à la partie de travail et une extrémité proximale (44) reliée à la poignée (32), où la tige (12) peut tourner par rapport à la poignée (32) autour d'un axe longitudinal (30) de la tige (12), où la partie de travail (24) et l'élément allongé (42) sont solidaires en rotation par rapport à la poignée (32) et la tige (12) peut tourner par rapport à la partie de travail (24) et à l'élément allongé (42), **caractérisé en ce que** l'élément allongé (42) est relié de manière amovible à la poignée (32), et configuré pour une liaison solidaire en rotation avec la poignée (32) dans au moins deux orientations angulaires autour de l'axe longitudinal (30), où l'élément allongé (42) a une partie de liaison (84) pour une liaison avec la poignée (32), et la poignée (32) a une partie de réception (86), dans laquelle la partie de liaison (84) est insérée et maintenue solidaire en rotation par rapport à la poignée (32).

2. Instrument de la revendication 1, **caractérisé en ce que** la partie de liaison (84) peut être insérée dans la partie de réception (86) et retirée de celle-ci dans une direction transversale à l'axe longitudinal (30).

3. Instrument de la revendication 1 ou 2, **caractérisé en ce que** la partie de liaison (84) a une section transversale à l'axe longitudinal (30) qui est de forme polygonale, et la partie de réception (86) a une section transversale qui est de forme polygonale.

4. Instrument de la revendication 3, **caractérisé en ce que** la forme polygonale de la section transversale de la partie de liaison (84) a un nombre pair de bords, et où la forme polygonale de la section transversale de la partie de réception (86) est rectangulaire.

5. Instrument de l'une des revendications 1 à 4, **caractérisé en ce que** la partie de travail (24) a au moins un élément de travail mobile (28), la poignée (32) a au moins une partie de préhension mobile (36), et où l'élément allongé (42) est un élément de transmission de force qui est mobile par rapport à la tige (12) dans la direction de l'axe longitudinal (30) et relié de manière fonctionnelle à l'au moins un élément de travail mobile (28) et à l'au moins une partie de préhension mobile (36).

6. Instrument de l'une des revendications 1 à 5, **caractérisé en ce que** l'extrémité proximale (66) de la partie de travail (24) est reliée à l'extrémité distale (14) de la tige (12) de sorte que la partie de travail (24) est immobile par rapport à la tige (12) dans la direction de l'axe longitudinal (30), mais la tige (12) peut tourner autour de l'axe longitudinal (30) par rapport à la partie de travail (24).

7. Instrument de la revendication 6, **caractérisé en ce que** l'extrémité proximale (66) de la partie de travail (24) a un premier épaulement radial (80) s'étendant dans une direction circonférentielle, l'extrémité distale (14) de la tige (12) a un deuxième épaulement radial (76) s'étendant dans une direction circonférentielle, où le premier épaulement (80) et le deuxième épaulement (76) sont essentiellement complémentaires l'un par rapport à l'autre et s'engagent l'un avec l'autre.
